(19) Europäisches Patentamt
European Patent Office
Office européen des brevets

(11) Publication number: 0 193 249
A2

(12) EUROPEAN PATENT APPLICATION

(21) Application number: 86200300.1

(22) Date of filing: 27.02.86

(51) Int. Cl.[4]: C 07 C 127/22
C 07 D 285/00, C 07 D 239/42
C 07 D 213/81, C 07 D 241/24
A 61 K 31/17

(30) Priority: 01.03.85 NL 8500572

(43) Date of publication of application:
03.09.86 Bulletin 86/36

(84) Designated Contracting States:
AT BE CH DE FR GB IT LI LU NL SE

(71) Applicant: DUPHAR INTERNATIONAL RESEARCH B.V
C.J. van Houtenlaan 36
NL-1381 CP Weesp(NL)

(72) Inventor: Brouwer, Marius S.
c/o OCTROOIBUREAU ZOAN B.V. P.O. Box 140
NL-1380 AC Weesp(NL)

(72) Inventor: Van Hes, Roelof
c/o OCTROOIBUREAU ZOAN B.V. P.O. Box 140
NL-1380 AC Weesp(NL)

(74) Representative: Muis, Maarten et al,
OCTROOIBUREAU ZOAN B.V. P.O. Box 140
NL-1380 AC Weesp(NL)

# (54) Benzoyl urea derivatives having ati-tumor activity.

(57) The present invention relates to a group of new benzoyl urea derivatives of the formula

$$R_1\text{-}Z\text{-}R_2$$

having a better antitumor activity in comparison with known benzoyl urea derivatives. The compounds have been tested in several in vitro test models with different types of tumors.

EP 0 193 249 A2

DIR 0363

BENZOYL UREA DERIVATIVES HAVING ANTITUMOR ACTIVITY.

The invention relates to new benzoyl urea derivatives having antitumor activity, to the preparation of such compounds, and to compositions which comprise at least one of the new compounds as the active substance.

From United States Patent Specification 3,748,356 it is known that a group of 1-benzoyl-3-phenyl urea compounds has insecticidal properties. This insecticidal activity is based on a mechanism which has not yet been fully clarified. What is clear is that the insects are killed in that the said urea derivatives inhibit the chitin formation.

It is known from European Patent Application 83201263.7 (publ. no. 0107214) that a number of 1-benzoyl--3-phenyl urea compounds having insecticidal properties, and metabolites thereof have a cytostatic and tumoricidal activity on tumors in mammals.

Furthermore it is known from European Patent application 0.025.363 that some N-benzoyl-N'-pyridyloxy phenyl urea compounds also have an antitumor activity.

It was found now that 1,3-substituted urea compounds of the formula

$$R_1-Z-R_2 \qquad (1)$$

wherein:

$R_1$ is an aryl group which may be substituted with a) 1-5 halogen atoms, b) 1-3 groups of the formula -O-A, wherein A is a hydrogen atom, an alkyl group having 1-3 C-atoms, and alkanoyl group having 1-6 C-atoms, a carbamoyl group optionally substituted with alkyl having 1-6 C-atoms or with phenyl, c) 1-2 amino groups which may be substituted with an alkanoyl group having 1-3 C-atoms, or with 1 or 2 alkyl groups

having 1-3 C-atoms which can form a ring together with the nitrogen atom, d) 1-2 dialkylamino N-oxyde groups, e) 1-2 alkylmercapto groups having 1-3 C-atoms, f) an alkylsulphinyl group or alkylsulphonyl group having 1-3 C-atoms, g) 1 or 2 alkyl groups having 1-6 C-atoms optionally substituted with halogen, h) a nitro group, i) a condensed hetero ring system, or j) a sulfonamide group, or wherein $R_1$ is a heteroaryl group which comprises as hetero atoms an oxygen atom, a sulphur atom and/or 1-3 nitrogen atoms, or the group N→O, which heteroaryl group may be substituted with phenyl, cycloalkyl having 3-10 C-atoms, 1-2 halogen atoms, hydroxyl, nitro, or optionally halogen-substituted alkyl having 1-6 C-atoms, or $R_1$ is a styryl group or aralkyl group, the alkyl group of which comprises 1-3 C-atoms and the aryl group may be substituted with 1-2 halogen atoms, 1-3 groups -O-A wherein A has the above-mentioned meaning, or 1-2 optionally halogen-substituted alkyl groups having 1-18 C-atoms, or a cycloalkyl group having 3-10 C-atoms, or $R_1$ is an amino group optionally substituted with 1 or 2 alkyl groups having 1-6 C-atoms, in which the alkyl group(s) together with the nitrogen atom can form a ring;

$R_2$ is an aryl group which may be substituted with a) 1-5 halogen atoms, b) 1-3 groups of the formula -O-A wherein A has the above-mentioned meaning, c) 1-2 optionally halogen-substituted alkyl groups having 1-6-C-atoms, d) cycloalkyl having 3-10 C-atoms, e) nitro, f) cyano, g) hydroxycarbonyl, h) alkoxycarbonyl having 1-6 C-atoms, i) alkanoyl having 1-3 C-atoms, j) 1-2 amino groups optionally substituted with 1 or 2 alkyl groups having 1-3 C-atoms, k) the group $-C(CF_3)_2OH$, or l) an alkoxy group having 1-3 C-atoms and optionally substituted with halogen

or pyridyl, or $R_2$ is a heteroaryl group which comprises an oxygen atom or a sulphur atom or 1-3 nitrogen atoms and which may be substituted with a) optionally halogen-substituted alkyl having 1-3 C-atoms, b) cycloalkyl having 3-10 C-atoms, c) 1-2 halogen atoms, d) phenyl or e) phenoxy, or $R_2$ is an aralkyl group the alkyl group of which comprises 1-3 C-atoms and the aryl group may be substituted with a) 1-2 halogen atoms, b) 1-3 groups -O-A, wherein A has the above-mentioned meaning, c) 1-2 alkyl groups having 1-3 C-atoms, or $R_2$ is an optionally halogen-substituted alkyl group having 1-6 C-atoms, a cycloalkyl group having 3-10 C-atoms or an amino group optionally substituted with 1 or 2 alkyl groups having 1-3 C-atoms,

Z is a group of the formula

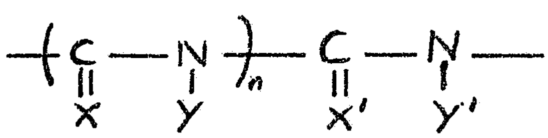

(2)

or a group of the formula

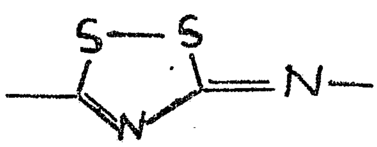

(3)

(with $R_1$ left and $R_2$ right), wherein X and X' are an oxygen atom or a sulphur atom, an amino group, a mono- or dialkylamino group (in this latter case Y is absent on the adjacent nitrogen atom and a double bond is present between that nitrogen atom and the carbon atom to which the dialkyl amino group is bonded) having 1-3 C-atoms per alkyl group, Y and Y' are a hydrogen atom or an optionally halogen-substituted alkyl group having 1-3 C-atoms, and <u>n</u> has the value 1 or 2,

have a very strong antitumor activity.

The invention does not relate to those compounds of the above formula (1), in which

$R_1$ is a group 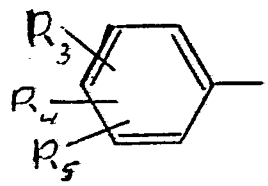 , $R_2$ is a group 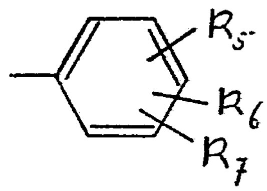

Y and Y' are a hydrogen atom, X and X' are an oxygen atom or a sulphur atom, and n has the value 1, in which groups

$R_3$ is a hydrogen atom, a group of the formula $-(-O-A)_p$, wherein A is a hydrogen atom, an alkanoyl group having 1-6 C-atoms, a carbamoyl group optionally substituted with alkyl having 1-6 C-atoms or with phenyl, or an alkyl mercapto group having 1-3 C-atoms,

$R_4$ is a hydrogen atom or a halogen atom, a nitro group, or an alkyl group optionally substituted with halogen and having 1-6 C-atoms,

$R_5$ is a hydrogen atom or halogen atom, a nitro group, an alkyl group optionally substited with halogen and having 1-6 C-atoms, an alkoxy group having 1-3 C-atoms, an amino group which may be substituted with 1 or 2 alkyl groups having 1-3 C-atoms,

$R_6$ is a hydrogen atom or a group $-(-O-A)_q$, wherein A is a hydrogen atom, an alkanoyl group having 1-6 C-atoms, a carbamoyl group optionally substituted with alkyl having 1-6 C-atoms or with phenyl, and

$R_7$ is a hydrogen atom or 1-3 halogen atoms, and p + q has the value 1-6. These compounds are disclosed in non-prepublished European Patent Application 84201221.3.

Nor does the invention relate to the compounds

1) 1-(2,6-difluorobenzoyl)-3-(2-hydroxy-4-chlorophenyl)-urea,

2) 1-(2,6-difluorobenzoyl)-3-(3-hydroxy-4-chlorophenyl)-urea,
3) 1-(2,6-difluorobenzoyl)-3-(4-hydroxyphenyl)urea,
4) 1-(2,6-difluorobenzoyl)-3-(2-hydroxy-4-trifluoromethylphenyl)urea,
5) 1-(2-chlorobenzoyl)-3-(2-hydroxy-4-trifluoromethoxyphenyl)urea,
6) 1-(2-chlorobenzoyl)-3-(2-hydroxy-4-trifluoromethylphenyl)urea,
7) 1-(2,6-difluorobenzoyl)-3-(4-chlorophenyl)urea,
8) 1-(2,6-difluorobenzoyl)-3-(4-trifluoromethylphenyl)-urea,
9) 1-(2-chlorobenzoyl)-3-(4-chlorophenyl)urea,
10) 1-(2-chlorobenzoyl)-3-(4-trifluoromethoxyphenyl)urea,
11) 1-(2,6-difluorobenzoyl)-3-(4-cyanophenyl)urea,
12) 1-(2,6-difluorobenzoyl)-3-[4-(1,1,2,3,3,3-hexafluoro-propoxy)phenyl]urea,
13) 1-(2,6-difluorobenzoyl)-3-(4-cyclohexylphenyl)urea,
14) 1-(2,6-difluorobenzoyl)-3-(4-trifluoromethoxyphenyl)-urea,
15) 1-(2,6-difluorobenzoyl)-3-(3,4-dichlorophenyl)urea,
16) 1-(2-chlorobenzoyl)-3-(4-trifluoromethylphenyl)urea,
17) 1-(2,6-dichlorobenzoyl)-3-(4-chlorophenyl)urea,
18) 1-(2,6-dichlorobenzoyl)-3-(4-trifluoromethylphenyl)-urea,
19) 1-benzoyl-3-(4-chlorophenyl)urea.

which belong to a group of compounds known from the above-mentioned European Patent Application 83201263.7 (publ. no. 0107214). In comparison with these known compounds, the new compounds have a much stronger antitumor activity.

The compounds according to the invention can be obtained in a manner known for the synthesis of analogous compounds as described for example in Netherlands Patent Specification 160,809.

The compounds of formula (1), wherein Z is the group of formula (2), wherein X and X' are an oxygen atom or a

sulphur atom and Y is a hydrogen atom, n = 1 and the remaining symbols have the above-mentioned meanings, can be obtained, for example, by reacting a compound of the formula

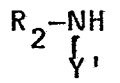 (4)

with a compound of the formula

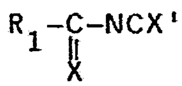 (5)

For preparing compounds of formula (1), wherein Z is a group of formula (2) in which Y is an alkyl group having 1-3 C-atoms and optionally substituted with halogen, said group Y can be introduced in a manner known *per se* by means of an alkylation reaction after completion of the above-described reaction of a compound of formula (4) with a compound of formula (5).

In an analogous manner the compounds of formula (1), wherein Z is a group of formula (2), n = 1, X' is an oxygen atom or a sulphur atom and Y' is a hydrogen atom, can be obtained by reaction of an amide of the formula

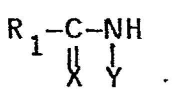 (6)

with a compound of the formula

$R_2-NCX'$ (7)

The corresponding compounds wherein Y' is an optionally halogen-substituted alkyl group having 1-3 C-atoms can be obtained by means of an alkylation reaction.

The compounds of formula (1), wherein X is an oxygen

atom or a sulphur atom, Y is hydrogen and n has the value 2, can be obtained in a similar manner by converting a compound of the formula

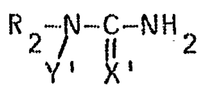

(8)

with a compound of the formula

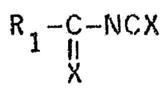

(9)

The above-described reactions are preferably carried out in the presence of an organic solvent, for example, an aromatic hydrocarbon, an alkyl halide, a non-cyclic or cyclic dialkyl ether, or acetonitrile, at temperatures between 0°C and the boiling-point of the solvent used.

Compounds of formula (1), wherein Z is the group of formula (3), can be obtained by oxidizing the corresponding dithiourea compounds to be obtained in the above- -described manner with, for example, a halogen, for example, bromine or iodine, as a result of which ring closure is effected via the two sulphur atoms. This reaction is preferably carried out in a solvent, for example, dichloroethane.

The compounds can be processed to compositions in the conventional manner.

A great advantage of the compounds according to the invention as compared with known cytostatics is that they have a low toxicity.

The antitumor activity of the compounds was determined in a number of in vitro test.

1) B16 melanoma test

B16 melanoma cells grow as a "monolayer". The doubling time of these cells in a cell culture is 12-16 hours. For carrying out the test, "6 multiwell" tissue culture plates

having a flat bottom with an area of 8 $cm^2$ proved to be best suitable.

On day 0, a quantity of 1.3 x $10^5$ B16 melanoma cells is introduced in each cell culture dish.

On day 1 the compounds to be examined are vortexed in the presence of glass beads. The compound to be tested is then added to the dishes with melanoma celles in the desired quantity. The incubation takes place at 37°C for 20 hours in a $CO_2$ incubator. The incubation is terminated by removing the culture medium with therein the compound to be tested, after which the cells are wahsed once and fresh medium is added.

Forty-eight hours after the beginning of the incubation the quantity of cells in each dish is measured by means of a microcell Coulter Counter. The results thus obtained are expressed by indicating the activity of the compounds by 1+, 2+, 3+, 4+ or 5+ having the following meanings:

1+: cell growth is inhibited by 1-20% with respect to the control test

2+: cell growth is inhibited by 21-40% with respect to the control test

3+: cell growth is inhibited by 41-60% with respect to the control test

4+: cell growth is inhibited by 61-80% with respect to the control test

5+: cell growth is inhibited by 81-100% with respect to the control test.

2a) Human cell line cytotoxicity

A number of representative compounds according to the invention were tested for cytotoxicity in a number of human tumor cell lines. The following cell lines have been used: bladder T24, malanoma IGR37, mammary MCF7, osteosarcoma A204, colon WIDR, HT29 and SW116 (P.P. Dendy and B.T. Hill, Human Tumour Drug, Sensititivty testing in

vitro, Academic Press 1983; H.B. Lamberts et al, Oncology 40, 301-304, (1983); A.A. van der Huizen, Aziridinyl cyclophosphazenes, synthesis, structure and cytostatic activity, thesis (1984), Groningen, the Netherlands).

Procedure:

Cells were seeded in 24-well cluster plates at a concentration of $10^5$ cells/ml of medium. After incubation for 24 hours at 37°C in an air/5% $CO_2$ atmosphere a suspension of the test compound in 0.5% CMC/saline was added to obtain a final concentration of 50 $\mu$g/ml.

Adriamycin was used as a positive control. After incubation for 72 hours in the presence of the test compound the cell layers were washed with phosphate bufferend saline and the cells were stained with the vital stain crystal violet. On the stained cell layer a visual assessment was done and the cell-inhibition scored:

4 approximately 100% cell growth inhibition
3 approximately 75% cell growth inhibition
2 approximately 50% cell growth inhibition
1 approximately 25% cell growth inhibition
0 no cell growth inhibition

The variation of the activity against the different cell lines has been indicated in tables A-E.

2b) Human cell lines clonogenic assay

Also a number of compounds was tested in this clonogenic assay (R. Ludwig et al, Cancer Chemother. Pharmacol. 12, 135-141, (1984); W.I. Scaefer, and K. Friend, Cancer Letters, 1, 259-262, (1976); P.P. Dendy, and B.T. Hill, Human Tumour Drug, Sensitivity testing in vitro, Academic Press 1983), using the following cell lines: mammary MCF7, and HTB26, colon WIDR and HTB38, lung HTB53, melanoma HTB66, and uterine HTB114.

Cisplatin, 5-fluorouracil, daunomycin, bleomycin and adriamycin were used with colon WIDR as comparisons.

Procedure:

Cells were seeded at the appropriate density for each cell line $10^2$-$10^5$ cells/dishes and incubated at 37°C in an air/5% $CO_2$ atmosphere. Compounds as solution or suspension in 0.5% CMC/saline were added during seeding or after an overnight pre-incubation period to a final concentration of: 50 $\mu$g/ml.

The duration of the treatment was about 3 times the cell replication cycle. Afterwards the assays were fixed, stained and scored for the presence of colonies. Colony formation is expressed as the number of colonies present on treated dishes as a percentage of the number of colonies on control dishes.

Sensitivity of B16 melanoma cells.

In the above-described manner, various compounds according to the invention were tested in dosages of 50 $\mu$g/ml in the form of an aqueous suspension. The results are recorded in the tables hereinafter:

TABLE A

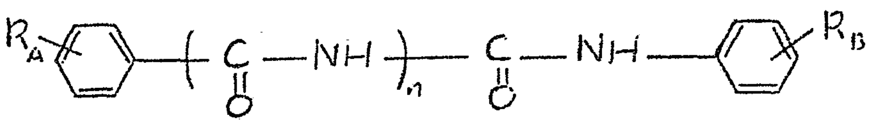

| Comp. no. | $R_A$ | $R_B$ | n | Melt. p. (°C) | Activity test 1 | Activity test 2a |
|---|---|---|---|---|---|---|
| 1 | 2Cl, 6-OH | 2,3,4,5,6-pentafluoro | 1 | 180 | 4+ | - |
| 2 | 2,3,4,5,6-pentafluoro | 4-OH, 3-$CF_3$ | 1 | 203 | 5+ | 4-4 |
| 3 | 2-F, 6-$N(CH_3)_2$ | 4-Cl | 1 | 161 | 5+ | 0-4 |
| 4 | 2,6-difluoro | 4-$COH(CF_3)_2$ | 1 | 204 | 5+ | 3-4 |
| 5 | " | 4-$COOC_2H_5$ | 1 | 215 | 3+ | - |
| 6 | 2,3,4,5,6-pentafluoro | 2,3,4,5,6-pentafluoro | 1 | 202 | 5+ | 2-4 |
| 7 | " | 4-Cl | 1 | 202 | 4+ | 2-4 |
| 8 | 4-$NH_2$ | 2-$NH_2$ | 1 | 240 (decomp.) | 1+ | 2-4 |
| 9 | 2,6-dichloro, 3,5-diamino | 4-Cl | 1 | 220 (decomp.) | 2+ | - |
| 10 | 2-piperidino | 4-$CF_3$ | 1 | 125 | 1+ | - |
| 11 | 2-F, 6-$N(CH_3)_2$ | 4-$OCH_3$ | 1 | 153 | 5+ | 0-4 |
| 12 | 2F, 6-$N(CH_3)_2$ | 2,3,4,5,6-pentafluoro | 1 | 213 | 4+ | 0-0 |
| 13 | 2-$NH_2$ | 4-Cl | 1 | 208 | 1+ | 0-0 |
| 14 | 2,6-difluoro | 4-O-CH- 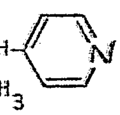 $CH_3$ | 1 | 188 | 4+ | - |
| 15 | 2-$N(CH_3)_2$ | 4-Cl | 1 | 160 | 5+ | 0-0 |
| 16 | 2Cl, 6-OH | 4-Cl | 2 | 210 | 4+ | - |
| 17 | 2F, 6-$N(C_2H_5)_2$ | 4-Cl | 1 | 134 | 2+ | 0-0 |
| 18 | 2,3,4,5,6-pentafluoro | 4-$COH(CF_3)_2$ | 1 | 180 | - | 4-4 |
| 19 | " | 3-$CF_3$ | 1 | 194 | - | 2-4 |
| 20 | " | 4-OH | 1 | 210 | - | 4-4 |
| 21 | 2-F, 6-$N(CH)_3)_2$ | 4-$OCH_3$ | 1 | 153 | - | 0-4 |

TABLE A (ctd.)

| Comp. no. | $R_A$ | $R_B$ | n | Melt. p. (°C) | Activity test 1 | Activity test 2a. |
|---|---|---|---|---|---|---|
| 22 | 2-Cl, 6-$N(CH_3)_2$ | 4-Cl | 1 | 188 | – | 0–4 |
| 23 | 2-$SCH_3$, 6-$N(CH_3)_2$ | 4-Cl | 1 | 220 | – | 2–4 |
| 24 | 2-F, 6-$\underset{\downarrow O}{N}(CH_3)_2$ | 4-Cl | 1 | 135 | – | 4–4 |
| 25 | 2-$OCH_3$, 6-$N(CH_3)_2$ | 4-Cl | 1 | 220 | – | 2–4 |
| 26 | 2-$OCH_3$, 6-$N(CH_3)_2$ | 4-$OCF_3$ | 1 | 181 | – | 2–4 |
| 27 | 2-$OCH_3$, 6-$N(CH_3)_2$ | 4-$CF_3$ | 1 | 200 | – | 2–4 |
| 28 | 2-$OCH_3$, 6-$N(CH_3)_2$ | 4-$SCH_3$ | 1 | 183 | – | 2–4 |
| 29 | 2-$OCH_3$, 6-$SCH_3$ | 4-$SCH_3$ | 1 | 203 | – | 1–4 |
| 30 | 2-$SCH_3$, 6-Cl | 4-Cl | 1 | 196 | – | 1–4 |
| 31 | 2,6-di-$SCH_3$ | 4-Cl | 1 | 216 | – | 0–4 |
| 32 | 2-$SCH_3$, 6-Cl | 4-$OCH_3$ | 1 | 183 | – | 2–4 |
| 33 | 2-$\underset{\downarrow O}{S}CH_3$, 6-Cl | 4-Cl | 1 | 210 | – | 3–4 |
| 34 | 2-$SCH_3$, 6-F | 4-Cl | 1 | 164 | – | 3–4 |
| 35 | 2,6-di-$SCH_3$ | 4-$SCH_3$ | 1 | 218 | – | 2–4 |
| 36 | 2-$SCH_3$, 6-Cl | 4-$\underset{H}{N}(\underset{\| O}{C}-CH_3)$ | 1 | 235 | – | 0–1 |
| 37 | 2-$SCH_3$, 6-Cl | 4-$COH(CF_3)_2$ | 1 | 176 | – | 2–4 |

TABLE B

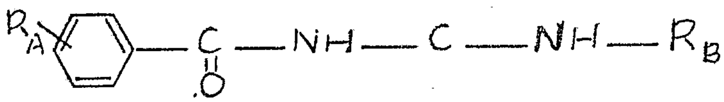

| Comp. no. | $R_A$ | $R_B$ | Melting p. (°C) | Activity | |
|---|---|---|---|---|---|
| | | | | test 1 | test 2a |
| 1 | 2-Cl, 6-O-$COCH_3$ | 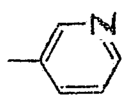 | 193 | 2+ | – |
| 2 | " | 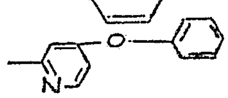 | 125 | 4+ | – |
| 3 | 2,6-dihydroxy | 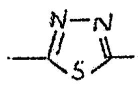adamantyl-1 | 170 | 2+ | – |
| 4 | 2-O-$COCH_3$ | 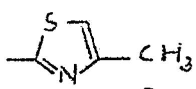 | 190 | 2+ | – |
| 5 | " | 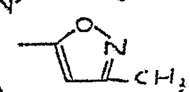 | 169 | 2+ | – |
| 6 | 2,6-dichloro | 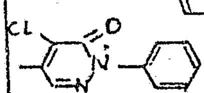 | 265 | 3+ | – |
| 7 | 2,6-difluoro | 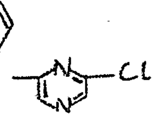 | 205 | 2+ | – |
| 8 | " | 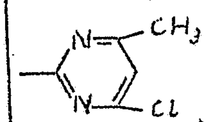 | 167 | 5+ | 0-4 |
| 9 | " | 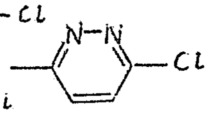 | >210 (decomp.) | 2+ | – |
| 10 | " | 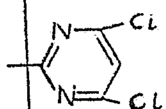 | 154 | 5+ | – |
| 11 | 2-Cl | 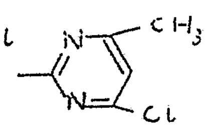 | 170 | 5+ | – |
| 12 | 2,6-difluoro | 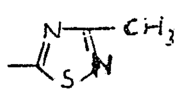 | 219 | 2+ | 0-0 |

TABLE B (ctd)

| Comp. no. | $R_A$ | $R_B$ | Melting p. (°C) | Activity test 1 | Activity test 2a |
|---|---|---|---|---|---|
| 13 | " | 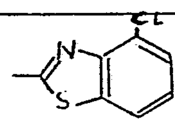 | 260 (decomp.) | 2+ | – |
| 14 | 2,6-difluoro | 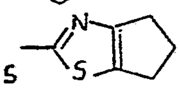 | 240 (decomp.) | 2+ | – |
| 15 | " | 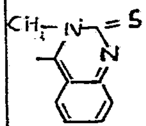 | >200 (decomp.) | 5+ | – |
| 16 | 2-Cl | 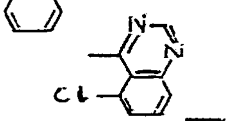 | 175 | 2+ | – |
| 17 | 2-Cl, 6-OH | 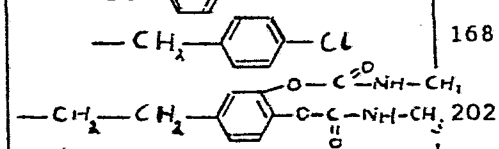 | 168 | 5+ | 0-2 |
| 18 | 2,6-dichloro | | 202 | 3+ | – |
| 19 | 2-Cl, 6-OH | cyclohexyl | 180 | 1+ | – |
| 20 | " | $n-C_4H_9-$ | 169 | 3+ | – |
| 21 | " | $-CH_2-CH_2-C_6H_5$ | 188 | 4+ | – |
| 22 | $2-O-COCH_3$ | 1-adamantyl | 166 | 4+ | – |
| 23 | 2,6-difluoro | $-(CH_2-CH_2Cl)_2$ | 126 | 3+ | – |
| 24 | " | $-N(CH_3)_2$ | 161 | 1+ | – |
| 25 | 2-F, $6-N(CH_3)_2$ | $n-C_4H_9-$ | 88 | 2+ | – |
| 26 | " | cyclohexyl | 157 | 3+ | – |

TABLE C

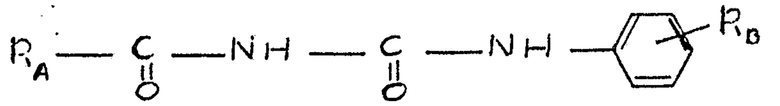

| Comp no. | $R_A$ | $R_B$ | Melting p. (°C) | Activity test 1 | Activity test 2a |
|---|---|---|---|---|---|
| 1 | 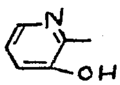 | 4-Cl | 206 | 4+ | – |
| 2 | 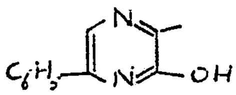 | 4-Cl | 285 | 2+ | – |
| 3 | 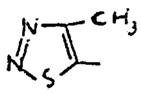 | 4-$OCF_2$-$CHF_2$ | 201 (decomp.) | 1+ | – |
| 4 | 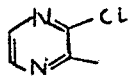 | 4-Cl | 229 | 5+ | – |
| 5 | 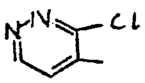 | 4-$CF_3$ | 174 | 5+ | – |
| 6 | 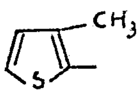 | 4-OH | 188 | 4+ | – |
| 7 | 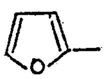 | 4-OH | 223 | 2+ | – |
| 8 | 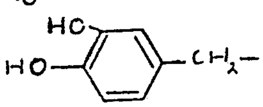 | 4-Cl | 205 | 4+ | – |
| 9 | $(CH_3)_3C$-$CH_2$- | 3,4-dihydroxy | 188 | 4+ | – |
| 10 | n-$C_{13}H_{27}$- | " | 163 | 5+ | 2-4 |
| 11 | cyclohexyl | " | 203 | 4+ | – |
| 12 | 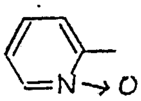 | 4-Cl | 191 | 5+ | 0-0 |
| 13 | 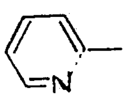 | 4-Cl | 164 | 4+ | 0-0 |
| 14 | 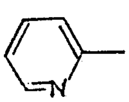 | 4-$CF_3$ | 189 | 4+ | – |

TABLE C (ctd)

| Comp no. | $R_A$ | $R_B$ | Melting p. (°C) | Activity | |
|---|---|---|---|---|---|
| | | | | test 1 | test 2a |
| 15 | 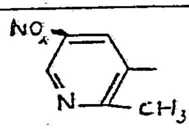 | 4-Cl | 192 | 1+ | - |
| 16 | 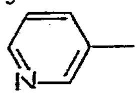 | 4-Cl | 260 | 1+ | - |
| 17 | 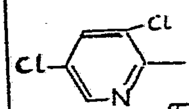 | 4-Cl | 199 | 4+ | - |
| 18 | 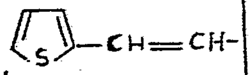 | 4-Cl | 246 | 5+ | - |
| 19 | 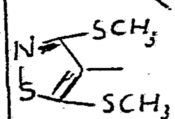 | 4-Cl | 184 | - | |

TABLE D

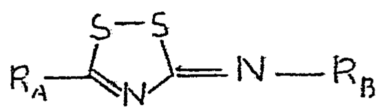

| Comp. no. | $R_A$ | $R_B$ | Melting p. (°C) | Activity | |
|---|---|---|---|---|---|
| | | | | test 1 | test 2a |
| 1 | 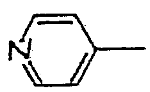 | $CH_3$ | 127 | 3+ | - |
| 2 | 2-chlorophenyl | $CH_3$ | 206 | 5+ | - |
| 3 | 2,6-difluorophenyl | $CH_3$ | 196 | 5+ | - |
| 4 | 2-methoxyphenyl | $CH_3$ | 186 | 5+ | - |
| 5 | 2,6-difluorophenyl | 4-chlorophenyl | 122 | 5+ | - |

TABLE E

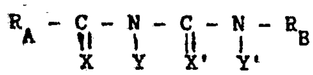

| Comp. no. | $R_A$ | X | Y | X' | Y' | $R_B$ | M.P. (°C) | Activity test 1 | Activity test 2a |
|---|---|---|---|---|---|---|---|---|---|
| 1 | 2,4-dichlorophenyl | O | H | S | H | 4-COOH-phenyl | 230 | 1+ | - |
| 2 | 2,6-difluorophenyl | S | H | S | H | 4-chlorophenyl | 190 | 5+ | 0-4 |
| 3 | " | O | H | S | H | " | 191 | 1+ | - |
| 4 | 2-chloro, 6-$SCH_3$phenyl | S | H | S | H | $CH_3$- | 149 | 4+ | - |
| 5 | 2,6-difluorophenyl | S | H | S | H | $CH_3$- | 110 | 3+ | - |
| 6 | 2Cl, 6-$SCH_3$phenyl | S | H | S | H | n-$C_4H_9$- | 102 | 5+ | - |
| 7 | 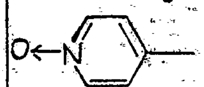 | S | H | S | H | $CH_3$- | 198 | 3+ | - |
| 8 | 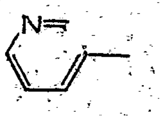 | S | H | S | H | 4-chlorophenyl | 184 | 5+ | - |
| 9 | 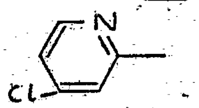 | S | H | S | H | n-$C_4H_9$ | 80 | 4+ | - |
| 10 | 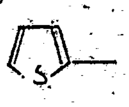 | S | H | S | H | 4-n-$C_4H_9$phenyl | 117 | 5+ | - |
| 11 | -$N(CH_3)_2$ | S | H | S | H | 2,6-difluorophenyl | 180 | 5+ | 4-4 |
| 12 | -$NH_2$.HCl | NH | H | NH | H | 4-chlorophenyl | 204 | 1+ | - |
| 13 | 2-methoxyphenyl | S | H | S | H | $C_2H_5$ | 77 | 4+ | - |
| 14 | 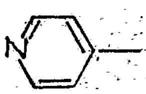 | S | H | S | H | $CH_3$- | 176 | 4+ | - |
| 15 | 4-$(CH_3)_2$N-phenyl | S | $CH_3$ | S | $CH_3$ | $CH_3$- | 116 | 5+ | - |
| 16 | n-$C_4H_9$ | S | H | S | H | phenyl | 69 | 5+ | 0-4 |
| 17 | cyclohexyl | S | H | S | H | $C_2H_5$- | 148 | 4+ | - |
| 18 | 2-Cl, 6-$CH_3$phenyl | S | H | S | H | n-$C_4H_9$ | 119 | 5+ | - |
| 19 | 2-Cl, 6-$CH_3$Sphenyl | S | H | S | H | 4-cyclohexylphenyl | 162 | 5+ | - |

TABLE E (ctd)

| Verb. no. | $R_A$ | X | Y | X' | Y' | $R_B$ | M.P. (°C) | Activity test 1 | Activity test 2a |
|---|---|---|---|---|---|---|---|---|---|
| 20 | 2,6-difluorophenyl | S | H | S | H | $n-C_4H_9$ | 113 | 5+ | – |
| 21 | " | S | H | S | H | $4-n.C_4H_9$-phenyl | 127 | 5+ | – |
| 22 | 2-Cl, 6-$SO_2CH_3$phenyl | S | H | S | H | $n-C_4H_9$ | 210 | 5+ | – |
| 23 | 2-Cl, 6-$SCH_3$phenyl | S | H | S | H | 4-$C_2H_5$phenyl | 116 | 5+ | 2-4 |
| 24 | 2,6-difluorophenyl | S | H | S | H | $C_2H_5$- | 145 | 5+ | – |
| 25 | " | S | H | S | H | 4-$OCH_3$phenyl | 163 | 5+ | – |
| 26 | 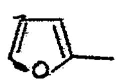 | S | H | S | H | $CH_3$- | 140 | 5+ | 1-4 |
| 27 | " | S | H | S | H | $C_2H_5$- | 95 | 4+ | – |
| 28 | 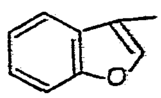 | S | H | S | H | $CH_3$- | 216 | 3+ | – |
| 29 | 2-$SCH_3$phenyl | O | H | S | H | 4-Clphenyl | 131 | 1+ | – |
| 30 | 2,6-difluorophenyl | $N(CH_3)_2$ | – | O | H | " | 130 | 5+ | – |
| 31 | 2,3,4,5,6-penta-fluorophenyl | O | H | S | H | 4-chlorophenyl | 276 | – | 4-4 |
| 32 | 2F, 6-$N(CH_3)_2$phenyl | S | H | S | H | " | 149 | – | 0-3 |
| 33 | 2-hydroxyphenyl | S | H | S | H | " | 207 | – | 2-4 |
| 34 | " | O | H | S | H | " | 185 | – | 3-4 |
| 35 | 3,4-dihydroxyphenyl | S | H | S | H | " | 196 | – | 4-4 |

As stated hereinbefore, the compounds according to the invention have a much stronger activity than the known benzoylurea derivatives having antitumor activity which are described in European Patent application 83201263.7. In Table F below, the activity is recorded of a number of known compounds, tested in a concentration which is 10 times as high, i.e. 500 /ug/ml:

TABLE F

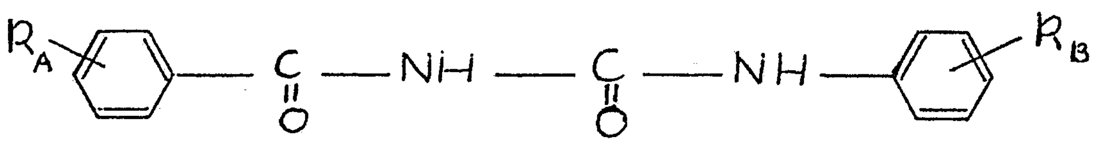

| Comp. no. | $R_A$ | $R_B$ | Activity (500 /ug/ml) |
|---|---|---|---|
| 1 | 2,6-difluoro | 4-Cl | 1+ |
| 2 | 2-Cl | 4-Cl | – |
| 3 | 2-Cl | 4-$OCF_3$ | – |
| 4 | 2,6-difluoro | 4-Cl, 2-OH | – |
| 5 | " | 4-Cl, 3-OH | 2+ |
| 6 | 2-Cl | 2-OH, 4-$OCF_3$ | – |
| 7 | 2,6-difluoro | 4-CN | – |
| 8 | 2-Cl | 4-$CF_3$ | – |
| 9 | 2,6-dichloro | 4-Cl | – |
| 10 | 2,6-difluoro | 2-OH, 4-$CF_3$ | – |
| 11 | 2-Cl | 2-OH, 4-$CF_3$ | – |
| 12 | 2,6-difluoro | 3-Cl, 4-O-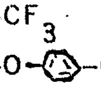-Cl | 3+ |

The antitumor activity of the compounds determined in vitro is confirmed by the preliminary results obtained in an in vivo test in mice.

The preparation of the compounds will be described in greater detail with reference to the ensuing specific examples.

EXAMPLE I

1-(2,3,4,5,6-pentafluorobenzoyl)-3-(2,3,4,5,6-pentafluorophenyl)urea

To a solution of 0.92 g of pentafluoroaniline in 25 ml of dry ether an equivalent quantity (1.2 g) of pentafluorobenzoyl-isocyanate was added at room temperature. After stirring for 2 hours the formed precipitate was sucked off. Yield 1.4 g (70%) of the above-mentioned compound. Melting-point 202°C.

EXAMPLE II

1-(4-aminobenzoyl)-3-(2-aminophenyl)urea.

In the manner described in Example I the compound 1--(4-nitrobenzoyl-3-(2-nitrophenyl)urea was prepared starting from 2-nitroaniline and 4-nitrobenzoylisocyanate. 11 g Of this compound were dissolved in 200 ml of dimethyl formamide and recuced in a Parr apparatus by means of 10 g of Raney nickel at a hydrogen pressure of 2-3 atm. After the reaction the solution was filtered off and evaporated under reduced pressure. The residue was recrystallized from a mixture of acetone and isopropanol. Yield 7.1 g (79%) of the title compound. Melting-point 240°C (decomposition).

EXAMPLE III

1-Cyclohexylcarbonyl-3-(3,4-didhyroxyphenyl)urea.

A solution of 1.4 g of cyclohexane carboxamide and 2.1 g of 3,4-dimethoxyphenylisocyanate in 20 ml of dry xylene was refluxed for 16 hours. After cooling to room temperature the formed precipitate was filtered off and wahsed with hexane. Yield 3.3 g (91%) of 1-cyclohexylcarbonyl-3--(3,4-dimethoxyphenyl)urea having a melting-point of 174°C.

2.0 g Of the compounds thus obtained were dissolved in 30 ml of dichloromethane. The solution was cooled to -60°C under an atmosphere of nitrogen. A solution of 10 ml of boron tribromide in 25 ml of dichloromethane was added dropwise. Stirring for 16 hours at room temperature was then carried out. The reaction mixture was cooled to -50°C and decomposed with methanol/water. After extracting with dichloromethane, drying, evaporating and recrystallizing from acetone/isopropyl ether, 1.3 g (72%) of the title compound were obtained. Melting-point 203°C.

EXAMPLE IV

1-(2-Pyridylcarbonyl)-3-(4-chlorophenyl)urea was obtained according to the method of Example I. 0.75 g Of this compound was dissolved in 5 ml of acetic acid and 1 ml of hydrogen peroxide (35%). The mixture was heated on a steam bath and 1.5 ml of hydrogen peroxid were added again after 2 and 3 hours. After 4.5 hours on the steam bath the mixture was cooled. The crystalline substance was sucked off and washed with isopropanol. Yield 0.3 g (38%) of the corresponding N-oxide. Melting-point 191°C.

EXAMPLE V

1.0 g Of 2,6-difluoro-N,N-dimethylbenzamidine.HCl was dissolved in 15 ml of dimethyl formamide. 0.6 ml Of triethylamine and 0.73 g of 4-chlorophenyl isocyanate were added. After stirring at room temperature for 24 hours, the reaction mixture was poured in water and extracted with dichloromethane. In this manner 1.24 g (81%) of compound No. 30 from Table E were obtained with a melting-point of 130°C.

EXAMPLE VI

3.3 g Of 2-methoxythiobenzamide and 1.7 g of ethylisothiocyanate were dissolved in 50 ml of acetonitrile. After cooling in ice water, 0.9 g of 55% sodium hydride in oil

were added to the mixture. Stirring at room temperature for 3 hours, filtering, acidifying with acetic acid, adding water and sucking off was carried out. After recrystallization from isopropanol, 2.7 g (53%) of 1-(2-methoxythiobenzoyl)-3-ethylthiourea were obtained. Melting-point 77°C.

EXAMPLE VII

1.3 g Of 1-(2-chlorothiobenzoyl)-3-methylthiourea were dissolved in 25 ml of dichloroethane. A solution of 0.27 ml of bromine in 10 ml of dichloroethane was added dropwise. After stirring for 30 minutes the formed precipitate was filtered off and suspended in acetonitrile. 1.3 ml Of triethylamine were added. After stirring for 1 hour, adding water, filtering off and recrystallizing from isopropanol, 0.4 g (31%) of compound no. 2 from Table D was obtained. Melting-point 206°C.

EXAMPLE VIII

4.3 g Of 2-chloro-6-benzyloxybenzoylisocyanate were dissolved in 15 ml of acetonitrile. 2.55 g Of 4-chlorophenylurea were added to this solution. The mixture was refluxed for 1.5 hours. After cooling, sucking off the solid and washing with ether, 4.4 g (64%) of 1-(2-chloro-6-benzyloxybenzoyl)-5-(4-chlorophenyl)biurete were obtained with a melting-point of 166°C.

1.0 g Of this compound was dissolved in 5 ml of 45% HBr/glacial acetic acid. The mixture was heated at 55°C for 1 hour. After cooling, pouring in water, filtering off the solid and recrystallizing from acetone/acetonitrile, 0.68 g (85%) of 1-(2-chloro-6-hydroxybenzoyl)-5-(4-chlorophenyl) biurete was obtained. Melting-point 210°C.

EXAMPLE IX

To a cooled solution of 0.8 g of 1-(2-fluoro-6-methoxybenzoyl)-3-(4-trifluoromethylphenyl)urea in a casius-tube 1,5 ml of dimethylamine is added. After 8-12 hours at 100°C, the cooled contents of the tube is pourred in ice-water and the precipitate is collected and washed with water. Yield: 0.6 g of 1-(2-dimethylamino-6-methoxybenzoyl)-3-(4-trifluoromethylphenyl)urea having a melting-point of 200°C.

CLAIMS:

1. Compositions having antitumor activity and comprising a urea derivative as the active substance, characterized in that they comprise at least one compound of the formula

$$R_1-Z-R_2 \qquad (1)$$

wherein:

$R_1$ is an aryl group which may be substituted with a) 1-5 halogen atoms, b) 1-3 groups of the formula -O-A, wherein A is a hydrogen atom, an alkyl group having 1-3 C-atoms, and alkanoyl group having 1-6 C-atoms, a carbamoyl group optionally substituted with alkyl having 1-6 C-atoms or with phenyl, c) 1-2 amino groups which may be substituted with an alkanoyl group having 1-3 C-atoms, or with 1 or 2 alkyl groups having 1-3 C-atoms which can form a ring together with the nitrogen atom d) 1-2 dialkylamino N-oxyde groups, e) 1-2 alkylmercapto group having 1-3-C-atoms, f) an alkylsulphinyl group or alkylsulphonyl group having 1-3 C-atoms, g) 1 or 2 alkyl groups having 1-6 C-atoms optionally substituted with halogen, h) a nitro group, i) a condensed hetero ring system, or j) a sulfonamide group, or wherein $R_1$ is a heteroaryl group which comprises as hetero atoms an oxygen atom, a sulphur atom and/or 1-3 nitrogen atoms, or the group N→O, which heteroaryl group may be substituted with phenyl, cycloalkyl having 3-10 C-atoms, 1-2 halogen atoms, hydroxyl, nitro, or optionally halogen-substituted alkyl having 1-6 C-atoms, or $R_1$ is a styryl group or aralkyl group, the alkyl group of which comprises 1-3 C-atoms and the aryl group may be substituted with 1-2 halogen atoms, 1-3 groups -O-A wherein A has the above-mentioned meaning, or 1-2 optionally halogen-substituted alkyl groups having 1-18 C-atoms, or a cycloalkyl

group having 3-10 C-atoms, or $R_1$ is an amino group optionally substituted with 1 or 2 alkyl groups having 1-6 C-atoms, in which the alkyl group(s) together with the nitrogen atom can form a ring;

$R_2$ is an aryl group which may be substituted with a) 1-5 halogen atoms, b) 1-3 groups of the formula -O-A wherein A has the above-mentioned meaning, c) 1-2 optionally halogen-substituted alkyl groups having 1-6-C-atoms, d) cycloalkyl having 3-10 C-atoms, e) nitro, f) cyano, g) hydroxycarbonyl, h) alkoxycarbonyl having 1-6 C-atoms, i) alkanoyl having 1-3 C-atoms, j) 1-2 amino groups optionally substituted with 1 or 2 alkyl groups having 1-3 C-atoms, k) the group $-C(CF_3)_2OH$, or l) an alkoxy group having 1-3 C-atoms and optionally substituted with halogen or pyridyl, or $R_2$ is a heteroaryl group which comprises an oxygen atom or a sulphur atom or 1-3 nitrogen atoms and which may be substituted with a) optionally halogen-substituted alkyl having 1-3 C-atoms, b) cycloalkyl having 3-10 C-atoms, c) 1-2 halogen atoms, d) phenyl or e) phenoxy, or $R_2$ is an aralkyl group the alkyl group of which comprises 1-3 C-atoms and the aryl group may be substituted with a) 1-2 halogen atoms, b) 1-3 groups -O-A. wherein A has the above-mentioned meaning, c) 1-2 alkyl groups having 1-3 C-atoms, or $R_2$ is an optionally halogen-substituted alkyl group having 1-6 C-atoms, a cycloalkyl group having 3-10 C-atoms or an amino group optionally substituted with 1 or 2 alkyl groups having 1-3 C-atoms,

Z is a group of the formula

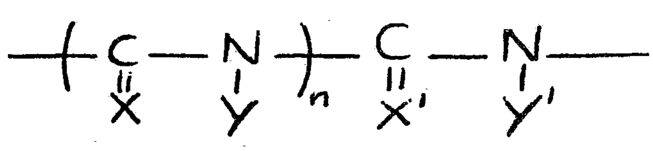

(2)

or a group of the formula

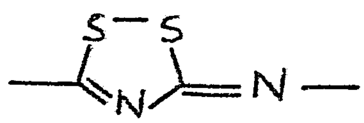

(3)

(with $R_1$ left and $R_2$ right), wherein X and X' are an oxygen atom or a sulphur atom, an amino group, a mono- or dialkylamino group (in this latter case Y is absent on the adjacent nitrogen atom and a double bond is present between that nitrogen atom and the carbon atom to which the dialkyl amino group is bonded) having 1-3 C-atoms per alkyl group, Y and Y' are a hydrogen atom or an optionally halogen-substituted alkyl group having 1-3 C-atoms, and n has the value 1 or 2, with the exclusion of the compounds in which

$R_1$ is a group 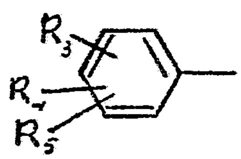 , $R_2$ is a group 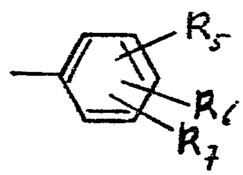

Y and Y' are a hydrogen atom, X and X' are an oxygen atom or a sulphur atom, and n has the value 1, in which groups

$R_3$ is a hydrogen atom, a group of the formula $-(-O-A)_p$, wherein A is a hydrogen atom, an alkanoyl group having 1-6 C-atoms, a carbamoyl group optionally substituted with alkyl having 1-6 C-atoms or with phenyl, or an alkyl mercapto group having 1-3 C-atoms,

$R_4$ is a hydrogen atom or a halogen atom, a nitro group, or an alkyl group optionally substituted with halogen and having 1-6 C-atoms,

$R_5$ is a hydrogen atom or halogen atom, a nitro group, an alkyl group optionally substited with halogen and having 1-6 C-atoms, an alkoxy group having 1-3

C-atoms, an amino group which may be substituted with 1 or 2 alkyl groups having 1-3 C-atoms,

$R_6$ is a hydrogen atom or a group $-(-O-A)_q$, wherein A is a hydrogen atom, an alkanoyl group having 1-6 C-atoms, a carbamoyl group optionally substituted with alkyl having 1-6 C-atoms or with phenyl, and

$R_7$ is a hydrogen atom or 1-3 halogen atoms, and p + q has the value 1-6,

and with the exclusion of the compounds:

1) 1-(2,6-difluorobenzoyl)-3-(2-hydroxy-4-chlorophenyl)-urea,
2) 1-(2,6-difluorobenzoyl)-3-(3-hydroxy-4-chlorophenyl)-urea,
3) 1-(2,6-difluorobenzoyl)-3-(4-hydroxyphenyl)urea,
4) 1-(2,6-difluorobenzoyl)-3-(2-hydroxy-4-trifluoromethylphenyl)urea,
5) 1-(2-chlorobenzoyl)-3-(2-hydroxy-4-trifluoromethoxyphenyl)urea,
6) 1-(2-chlorobenzoyl)-3-(2-hydroxy-4-trifluoromethylphenyl)urea,
7) 1-(2,6-difluorobenzoyl)-3-(4-chlorophenyl)urea,
8) 1-(2,6-difluorobenzoyl)-3-(4-trifluoromethylphenyl)-urea,
9) 1-(2-chlorobenzoyl)-3-(4-chlorophenyl)urea,
10) 1-(2-chlorobenzoyl)-3-(4-trifluoromethoxyphenyl)urea,
11) 1-(2,6-difluorobenzoyl)-3-(4-cyanophenyl)urea,
12) 1-(2,6-difluorobenzoyl)-3- 4-(1,1,2,3,3,3-hexafluoro-propoxy)phenyl urea,
13) 1-(2,6-difluorobenzoyl)-3-(4-cyclohexylphenyl)urea,
14) 1-(2,6-difluorobenzoyl)-3-(4-trifluoromethoxyphenyl)-urea,
15) 1-(2,6-difluorobenzoyl)-3-(3,4-dichlorophenyl)urea,
16) 1-(2-chlorobenzoyl)-3-(4-trifluoromethylphenyl)urea,
17) 1-(2,6-dichlorobenzoyl)-3-(4-chlorophenyl)urea,
18) 1-(2,6-dichlorobenzoyl)-3-(4-trifluoromethylphenyl)-urea,

19) 1-benzoyl-3-(4-chlorophenyl)urea.

2. A method of preparing compositions as claimed in Claim 1, characterized in that an active substance of formula 1 in which the symbols have the meaning given in Claim 1, are brough into a form suitable for medical application.

3. A method of treating and controlling tumors, characterized in that a composition as claimed in Claim 1 is used.

4. Compounds of formula (1), in which the symbols have the meanings given in Claim 1.

5. A method of preparing urea derivatives, characterized in that compounds as claimed in Claim 4 are prepared in a manner known for the synthesis of analogous compounds.

6. A method as claimed in Claim 5, characterized in that a compound of formula (1) is prepared, in which Z is a group of the formula

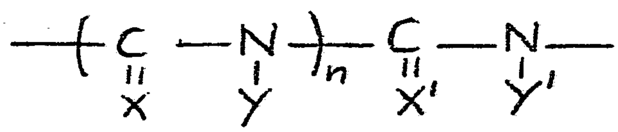

(2)

wherein X and X' are an oxygen atom or a sulphur atom, Y is a hydrogen atom, n has the value 1 and Y' has the meaning given in Claim 1, by converting a compound of the formula

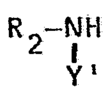

(4)

with a compound of the formula

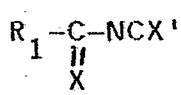

(5)

7. A method as claimed in Claim 5, characterized in that a compound of formula (1) is prepared, in which Z is

a group of formula (2), wherein n = 1, X' is an oxygen atom or a sulphur atom and Y is a hydrogen atom, and X has the meaning given in Claim 1, by converting a compound of the formula

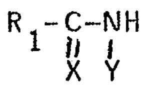 (6)

with a compound of the formula

$R_2$-NCX' (7)

8. A method as claimed in Claim 5, characterized in that a group Y and a group Y', respectively is introduced in a manner known *per se* by means of an alkylation reaction, in a compound obtained according to the method as claimed in Claim 6 or 7.

9. A method as claimed in Claim 5, characterized in that a compound of formula (1) is prepared in which Z is the group of formula (2), in which n = 2, X is an oxygen atom or a sulphur atom, and Y is a hydrogen atom, by converting a compound of the formula

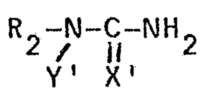 (8)

with a compound of the formula

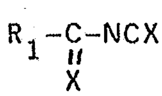 (9)

wherein $R_1$, $R_2$, X' and Y' have the meanings given in Claim 1.

10. A method as claimed in Claim 5, characterized in that a compound of formula (1) is prepared in which Z is a group of formula (3) by oxidizing a compound of the formula

$$R_1-\underset{\underset{S}{\|}}{C}-NH-\underset{\underset{S}{\|}}{C}-NH-R_2 \qquad (10)$$

wherein $R_1$ and $R_2$ have the meaning given in Claim 1.